# EUROPEAN PATENT SPECIFICATION

(11) **EP 4 123 306 B1**
(45) Date of publication and mention of the grant of the patent: **11.02.2026**
(21) Application number: 22186201.4
(22) Date of filing: 21.07.2022
(51) Int. Cl.: G01N 33/564, G01N 33/573

(54) **IMMUNOLOGICAL METHOD FOR DETECTING SPECIFIC ANTIBODIES IN CELIAC DISEASE**
MMUNOLOGISCHES VERFAHREN ZUM NACHWEIS VON MIT ZÖLIAKIE IN ZUSAMMENHANG STEHENDEN ANTIKÖRPERN
MÉTHODE IMMUNOLOGIQUE DE DÉTECTION D'ANTICORPS SPÉCIFIQUES D'UNE MALADIE COELIAQUE

(30) Priority: 23.07.2021 IT 202100019616
(43) Date of publication of application: 25.01.2023
(73) Proprietor: UNIVERSITA' DEGLI STUDI DI TRIESTE, 34127 Trieste (IT); Istituto di Ricovero E Cura a Carattere Scientifico Materno-Infantile Burlo Garofolo, 34137 Trieste (IT)
(72) Inventor: NOT, Tarcisio, TRIESTE (IT); DE LEO, Luigina, TRIESTE (IT)
(74) Representative: Delbarba, Andrea

(56) References cited:
- EP-A1- 1 978 364
- US-A1- 2009 305 303
- US-A1- 2013 196 336
- DE LEO LUIGINA ET AL: "Immunohistologic analysis of the duodenal bulb: a new method for celiac disease diagnosis in children", GASTROINTESTINAL ENDOSCOPY, ELSEVIER, NL, vol. 88, no. 3, 26 May 2018 (2018-05-26), pages 521 - 526, XP085446827, ISSN: 0016-5107, DOI: 10.1016/J.GIE.2018.05.014
- "Celiac Disease - From the Bench to the Clinic, Chapter 2", 30 January 2019, INTECHOPEN, ISBN: 978-1-78985-050-5, article WU HUAN ET AL: "Challenges with Point-Of-Care Tests (POCT) for Celiac Disease", XP055903601, DOI: 10.5772/intechopen.81874
- RAIVIO T. ET AL: "Self transglutaminase-based rapid coeliac disease antibody detection by a lateral flow method", ALIMENTARY PHARMACOLOGY AND THERAPEUTICS, VOL. 24, 1 July 2006 (2006-07-01), pages 147 - 154, XP055903608, Retrieved from the Internet <URL:https://pubmed.ncbi.nlm.nih.gov/16803613/> [retrieved on 20220321], DOI: 10.1111/j.1365-2036.2006.02957.x

## Description

### FIELD OF THE INVENTION

The present invention describes an immunological method for detecting the presence of the specific antibodies of celiac disease extracted from an intestinal biopsy sample obtained from an individual with a clinical suspicion of celiac disease.

### STATE OF THE ART

Celiac disease is a gluten-dependent autoimmune disease that may present with gastrointestinal symptoms such as diarrhoea, abdominal distension and/or extraintestinal symptoms such as retarded growth, anaemia, osteopenia, arthralgia and infertility. It affects 1-2% of the general population with higher percentages among the groups at risk such as first-degree relatives (8%) and individuals with autoimmune diseases (4-6%). The diagnosis can easily be performed by means of an assay of the anti-transglutaminase (anti-tTG) and anti-endomysium (EMA) antibodies in blood, which are the specific, universally sensitive markers used in clinical practice for the diagnosis of celiac disease.

However, in about 10% of individuals, the symptoms are more indefinite (recurrent abdominal pains) and the diagnosis remains dubious because, notwithstanding the positivity of the autoantibodies in the blood, the intestinal biopsy does not show the classical condition of villous atrophy characterising the disease. In these individuals the suspicion of celiac disease can be confirmed or disproven with certainty by looking for the autoantibodies in the intestinal mucosa, which is the site where these antibodies are produced.

Furthermore, the search for these antibodies in the intestinal mucosa is the diagnostic key to resolving symptomatic cases with a genetic predisposition to celiac disease, in which the autoantibodies are absent from the blood or show very low positive concentrations, such as to raise doubts about their actual diagnostic value.

At present these autoantibodies can be identified through two methods:
1. anti-tTG in the intestinal mucosa are identified by means of a complex direct immunofluorescence method, with double staining in frozen intestinal biopsy sections; the method requires equipment and a computer-aided fluorescence microscope to process the image of the double staining as well as expert personnel.
2. EMA are identified with the EMA-biopsy method, which requires laboratory equipment, expert personnel and a long incubation time (72 hours at 37°C) in the presence of soluble fractions of gliadin to favour EMA release. After 72 hours, the culture liquid is centrifugated and collected for analysis with the indirect immunofluorescence technique on thin monkey oesophagus sections.

De Leo Luigina and coworkers (Gastrointestinal Endoscopy, vol88, No. 3, 26 May 2016, pages 521-526) describes the use of 2 tests to detect celiac disease in children: a first test is an immunohistological method using an anti-tTG antibody for the detection of intestinal IgA anti-tTG deposits in unfixed frozen sections from bulb duodenal samples and distal duodenum specimens. The second test involves a serum test which detects IgA anti-tTG antibodies via immobilized human transglutaminase (ELISA assay from Eurospital).

In fact, there is a need for a reliable analytical method for determining the intestinal autoantibodies of celiac disease, one that is both fast and easy to implement, so that every gastroenterology unit is able to autonomously complete the procedure for the diagnosis of celiac disease in all the clinical and biological forms it presents itself in.

### SUMMARY OF THE INVENTION

The present invention relates to an in vitro method for assessing the presence of celiac disease in an individual. In one embodiment, said method comprises the following steps:
a) fragmenting at least one intestinal biopsy sample obtained from the individual,
b) contacting the at least one sample obtained from step (a) with at least one human transglutaminase,
c) detecting the binding of at least one antibody in the sample to at least one human transglutaminase,
where binding of at least one antibody to at least one human transglutaminase indicates that the individual is positive for celiac disease.

### BRIEF DESCRIPTION OF THE FIGURES

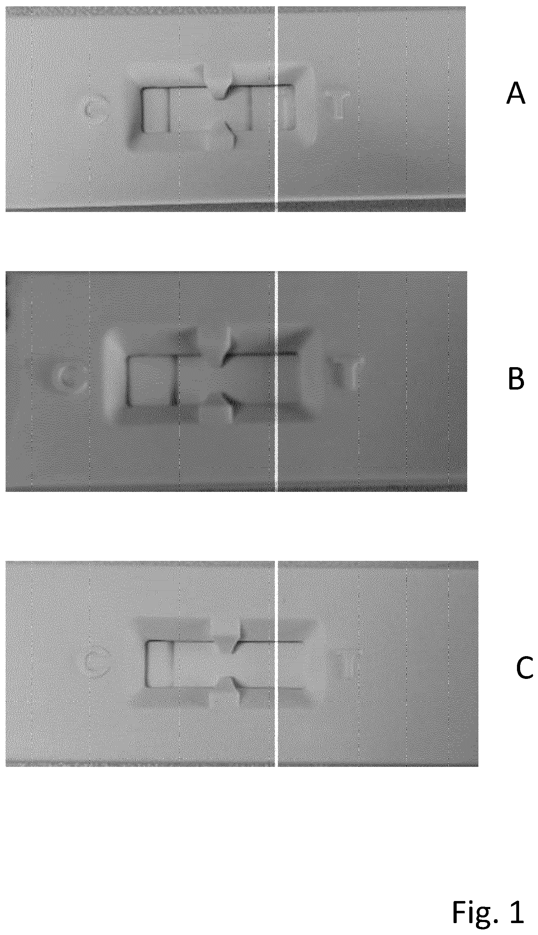

Figure 1 shows a test carried out with the method of the present invention where: the test gave a strongly positive result (Fig. 1 A, red line), a weakly positive result (1B, red line) and a negative result (1C, no red line) for intestinal anti-tTG.

### DETAILED DESCRIPTION OF THE INVENTION

The present invention relates to an in vitro method for assessing the presence of celiac disease in an individual. In one embodiment, said method comprises the following steps:
a) fragmenting at least one intestinal biopsy sample obtained from the individual,
b) contacting the at least one sample obtained from step (a) with at least one human transglutaminase,
c) detecting the binding of at least one anti-transglutaminase antibody in the sample with the at least one human transglutaminase,
where binding of at least one antibody to at least one human transglutaminase indicates that the individual is positive for celiac disease.

In one embodiment, said at least one human transglutaminase is transglutaminase type 2; said human transglutaminase type 2 corresponds to SEQ ID NO: 1 shown in Table 1.

Said at least one human transglutaminase is preferably used as a recombinant or purified/isolated protein.

Preferably, in this context, reference is made to use of the entire human transglutaminase protein, or homologues, analogues, variants, derivatives or fragments of human transglutaminase protein.

In a further embodiment of the invention, said at least one human transglutaminase is synthesised by means of conventional protein synthesis techniques known to the skilled person. For example, the protein can be synthesised by chemical synthesis using solid-phase peptide synthesis.

In a further embodiment of the invention, the human transglutaminase protein is isolated or purified with methods known to the person skilled in the art. For example, the human transglutaminase can be purified with chromatographic methods (gel filtration, ion exchange and immunoaffinity), by means of high-performance liquid chromatography (HPLC, RP-HPLC, ion exchange HPLC, size-exclusion HPLC) or by precipitation (immunoprecipitation). Alternatively, the human transglutaminase can be produced with DNA recombinant techniques known to the person skilled in the art.

**Table 1.**

| | | |
|---|---|---|
| SEQ ID NO. 1 | MAEELVLERCDLELETNGRDHHTADLCREKLVVRRGQPFWLTLHFEGRNYEASVDSLTFS VVTGPAPSQEAGTKARFPLRDAVEEGDWTATVVDQQDCTLSLQLTTPANAPIGLYRLSLE ASTGYQGSSFVLGHFILLFNAWCPADAVYLDSEEERQEYVLTQQGFIYQGSAKFIKNIPW NFGQFEDGILDICLILLDVNPKFLKNAGRDCSRRSSPVYVGRVVSGMVNCNDDQGVLLGR WDNNYGDGVSPMSWIGSVDILRRWKNHGCQRVKYGQCWVFAAVACTVLRCLGIPTRVVTN YNSAHDQNSNLLIEYFRNEFGEIQGDKSEMIWNFHCWVESWMTRPDLQPGYEGWQALDPT PQEKSEGTYCCGPVPVRAIKEGDLSTKYDAPFVFAEVNADVVDWIQQDDGSVHKSINRSL IVGLKISTKSVGRDEREDITHTYKYPEGSSEEREAFTRANHLNKLAEKEETGMAMRIRVG QSMNMGSDFDVFAHITNNTAEEYVCRLLLCARTVSYNGILGPECGTKYLLNLNLEPFSEK SVPLCILYEKYRDCLTESNLIKVRALLVEPVINSYLLAERDLYLENPEIKIRILGEPKQK RKLVAEVSLQNPLPVALEGCTFTVEGAGLTEEQKTVEIPDPVEAGEEVKVRMDLLPLHMG LHKLVVNFESDKLKAVKGFRNVIIGPA | Amino acid sequence of human transgluta minase type 2 |

In one embodiment, the at least one anti-transglutaminase antibody belongs to the IgA and/or IgG class. In other words, the method of the present invention makes it possible to detect the presence of at least one anti-transglutaminase antibody belonging to the IgA class and/or belonging to the IgG class. The method preferably enables the detection both of at least one antibody belonging to the IgA class and at least one antibody belonging to the IgG class.

In one embodiment, the at least one biopsy sample is obtained from a plurality of intestinal tissue samples from the individual, preferably from at least two samples of intestinal tissue in at least two different intestinal locations. The at least one biopsy sample is preferably obtained from the duodenal bulb and/or the distal duodenum.

In one embodiment of the invention, the sample obtained from step a) is contacted with recombinant human transglutaminase type 2 in an immunodiffusion assay. The immunodiffusion assay is selected from the immunodiffusion assays known to the person skilled in the art.

The immunodiffusion assay enables detection of the binding between the recombinant human transglutaminase type 2 and at least one anti-transglutaminase antibody in the sample by means of a visible colorimetric reaction. In other words, the presence of at least one anti-transglutaminase antibody in the sample gives rise to the formation of a visible immunoprecipitate that can be detected in step c). Detection takes place through observation and assessment of the presence of a precipitate. In one embodiment, in step c) detection of the binding of at least one antibody to the at least one human transglutaminase takes place by direct observation, i.e. by observation with the naked eye and/or with the aid of an instrument for detecting and/or quantifying a colorimetric reaction, known to the person skilled in the art.

Preferably, the immunodiffusion assay is capable of detecting the presence of at least one anti-transglutaminase antibody belonging to the IgA class and/or belonging to the IgG class in a single colorimetric reaction. In particular, the method is capable of detecting anti-human transglutaminase autoantibodies, i.e. antibodies produced by the same individual and which are capable of binding the transglutaminase type 2 of that individual.

In one embodiment, in step a) the sample is immersed in a salt solution, preferably a phosphate salt solution without calcium and magnesium, before being fragmented. Preferably, where in step a) the sample is fragmented for a time comprised between 3 and 10 minutes; more preferably it is fragmented to obtain a biological fluid. Preferably, the sample is mechanically fragmented using a pestle or another manual and/or automatic instrument known to the person skilled in the art.

The applicant has surprisingly discovered that the method described above in detail enables a simultaneous search for IgA and IgG anti-transglutaminase antibodies directly from intestinal biopsies, mainly owing to the use of human transglutaminase type 2 as an antigen to replace the monkey oesophagus tissue necessary for the EMA-biopsy method.

Furthermore, the method of the present invention enables a drastic reduction in the time it takes to determine these antibodies; in fact, in about 20 minutes it is possible to obtain the results of the intestinal anti-transglutaminase antibodies, compared to the over 72 hours of the EMA-biopsy test. Indeed, at the end of endoscopic examination, the method will already reveal whether the patient produces intestinal anti-transglutaminase antibodies and facilitates the diagnosis of celiac disease.

In fact, the mechanical fragmentation of the individual's sample, preferably at least one biopsy, will release the anti-transglutaminase antibodies without any need for a laboratory and equipment (e.g. thermostat, centrifuges), or long incubations and without the use of antigenic activators (gliadin fragments), which are by contrast necessary for the tests presently in use.

Furthermore, thanks to the use of at least two intestinal tissue samples from at least two different intestinal locations, one obtains a greater sensitivity of the method.

A second aspect of the present description relates to a kit (not part of the present invention) that comprises a carrier comprising at least one human transglutaminase. Preferably, said at least one human transglutaminase is transglutaminase type 2, preferably it is human transglutaminase type 2; said human transglutaminase type 2 corresponds to SEQ ID NO: 1 shown in Table 1. In a preferred embodiment, said carrier is made of a material selected from: cellulose, nitrocellulose and nylon; it is preferably made of nitrocellulose.

A third aspect of the present description relates to the use of the kit (not part of the present invention) described above in detail for in vitro detection of the presence of at least one anti-transglutaminase antibody.

### EXAMPLE

The present method rapidly identifies anti-transglutaminase antibodies of the IgA and IgG classes directly in intestinal biopsies immediately after they have been taken from the duodenal bulb and distal duodenum. In particular, the biopsy is immersed in 400 microlitres of Dulbecco's phosphate-buffered saline solution without calcium and magnesium at pH 7.3, and fragmented for 5 minutes with the aid of a pestle. At the end of fragmentation, a biological fluid is obtained.

Some drops of the biological fluid obtained, about 90 microlitres, were used to reveal the IgA and IgG anti-transglutaminase simultaneously by means of the rapid immunodiffusion assay, which gives a response in about 15 minutes. The rapid immunodiffusion test comprises human transglutaminase type 2 as an autoantigen.

Positivity is represented by a red line in the window T (Figure 1), whilst the blue line in the window C indicates the proper functioning of the test and for this reason must always be present. The results are read with the naked eye. The presence of a bright red line in the window T indicates positivity due to high concentrations of intestinal anti-tTG (Figure 1A), whereas a faint red line indicates a weak concentration of intestinal anti-tTG (Figure 1B). Intestinal anti-tTG have a high positive predictive value for the presence of celiac disease. The absence of lines in the window T (white background) indicates the absence of intestinal anti-tTG. The latter result has a high negative predictive value for the presence of celiac disease (Figure 1C).

## Claims

1. An in vitro method for assessing the presence of celiac disease in an individual, comprising the following steps:
(a) fragmenting at least one intestinal biopsy sample obtained from the individual,
(b) contacting the at least one sample obtained from step (a) with at least one human transglutaminase,
(c) detecting the binding of at least one antibody in the sample to at least one human transglutaminase,
where binding of at least one antibody to at least one human transglutaminase indicates that the individual is positive for celiac disease.

2. The method according to claim 1, wherein the at least one human transglutaminase is human transglutaminase type 2.

3. The method according to claim 1 or 2, wherein the at least one anti-transglutaminase antibody belongs to the IgA and/or IgG class.

4. The method according to any one of claims 1-3, wherein the at least one biopsy sample is obtained from at least two samples of intestinal tissue in at least two different intestinal locations.

5. The method according to any one of claims 1-4, wherein the at least one biopsy sample is obtained from the duodenal bulb and/or the distal duodenum.

6. The method according to any one of claims 1-5, wherein the sample obtained from step a) is contacted with the at least one protein in an immunodiffusion assay.

7. The method according to any one of claims 1-6, wherein in step a) the sample is immersed in a salt solution before being fragmented.

8. The method according to claim 7, wherein in step a) the sample is fragmented to obtain a biological fluid.

9. The method according to any one of claims 1-8, wherein in step a) the sample is mechanically fragmented.

## Patentansprüche

1. In-vitro-Verfahren zur Beurteilung des Vorliegens von Zöliakie bei einem Individuum, umfassend die folgenden Schritte:
(a) Fragmentieren mindestens einer von dem Individuum erhaltenen Darmbiopsieprobe,
(b) Inkontaktbringen der mindestens einen aus Schritt (a) gewonnenen Probe mit mindestens einer menschlichen Transglutaminase,
(c) Nachweis der Bindung mindestens eines Antikörpers in der Probe an mindestens eine humane Transglutaminase,
wobei die Bindung mindestens eines Antikörpers an mindestens eine humane Transglutaminase darauf hinweist, dass das Individuum positiv für Zöliakie ist.

2. Verfahren nach Anspruch 1, wobei die mindestens eine humane Transglutaminase humane Transglutaminase Typ 2 ist.

3. Verfahren nach Anspruch 1 oder 2, wobei der mindestens eine Anti-Transglutaminase-Antikörper zur Klasse IgA und/oder IgG gehört.

4. Verfahren nach einem der Ansprüche 1-3, wobei die mindestens eine Biopsieprobe aus mindestens zwei Proben von Darmgewebe an mindestens zwei verschiedenen Darmstellen gewonnen wird.

5. Verfahren nach einem der Ansprüche 1-4, wobei die mindestens eine Biopsieprobe aus dem Zwölffingerdarmwulst und/oder dem distalen Zwölffingerdarm gewonnen wird.

6. Verfahren nach einem der Ansprüche 1-5, wobei die aus Schritt a) gewonnene Probe mit dem mindestens einen Protein in einem Immundiffusionsassay in Kontakt gebracht wird.

7. Verfahren nach einem der Ansprüche 1-6, wobei in Schritt a) die Probe in eine Salzlösung getaucht wird, bevor sie fragmentiert wird.

8. Verfahren nach Anspruch 7, wobei in Schritt a) die Probe fragmentiert wird, um eine biologische Flüssigkeit zu erhalten.

9. Verfahren nach einem der Ansprüche 1-8, wobei in Schritt a) die Probe mechanisch fragmentiert wird.

## Revendications

1. Méthode in vitro d'évaluation de la présence d'une maladie cœliaque chez un individu, comprenant les étapes suivantes :
(a) fragmenter au moins un échantillon de biopsie intestinale prélevé sur l'individu,
(b) mettre en contact l'au moins un échantillon prélevé à l'étape (a) avec au moins une transglutaminase humaine,
(c) détecter la liaison d'au moins un anticorps dans l'échantillon à au moins une transglutaminase humaine,
où la liaison d'au moins un anticorps à au moins une transglutaminase humaine indique que l'individu est positif à la maladie cœliaque.

2. Méthode selon la revendication 1, dans laquelle l'au moins une transglutaminase humaine est une transglutaminase humaine de type 2.

3. Procédé selon la revendication 1 ou 2, dans laquelle l'au moins un anticorps anti-transglutaminase appartient à la classe des IgA et/ou des IgG.

4. Méthode selon l'une quelconque des revendications 1-3, dans laquelle l'au moins un échantillon de biopsie est obtenu à partir d'au moins deux échantillons de tissu intestinal prélevés dans au moins deux emplacements intestinaux différents.

5. Méthode selon l'une quelconque des revendications 1-4, dans laquelle l'au moins un échantillon de biopsie est prélevé du bulbe duodénal et/ou du duodénum distal.

6. Méthode selon l'une quelconque des revendications 1-5, dans laquelle l'échantillon prélevé à l'étape a) est mis en contact avec l'au moins une protéine dans un test d'immunodiffusion.

7. Méthode selon l'une quelconque des revendications 1-6, dans laquelle, à l'étape a), l'échantillon est immergé dans une solution saline avant d'être fragmenté.

8. Méthode selon la revendication 7, dans laquelle, à l'étape a), l'échantillon est fragmenté pour obtenir un fluide biologique.

9. Méthode selon l'une quelconque des revendications 1-8, dans laquelle, à l'étape a), l'échantillon est fragmenté mécaniquement.
